(19) Europäisches Patentamt
European Patent Office
Office européen des brevets

(11) Numéro de publication: **0 310 519 B1**

(12) # FASCICULE DE BREVET EUROPEEN

(45) Date de publication de fascicule du brevet: **19.08.92**

(51) Int. Cl.[5]: **C07J 41/00**, A61K 31/705

(21) Numéro de dépôt: **88402476.1**

(22) Date de dépôt: **30.09.88**

Le dossier contient des informations techniques présentées postérieurement au dépôt de la demande et ne figurant pas dans le présent fascicule.

(54) **Nouveaux esters d'acide androstane 17-carboxylique, procédé pour leur préparation et médicament les contenant.**

(30) Priorité: **02.10.87 FR 8713658**

(43) Date de publication de la demande: **05.04.89 Bulletin 89/14**

(45) Mention de la délivrance du brevet: **19.08.92 Bulletin 92/34**

(84) Etats contractants désignés: **AT BE CH DE ES FR GB GR IT LI LU NL SE**

(56) Documents cités:
**EP-A- 0 024 983**
**EP-A- 0 101 659**
**DD-C- 138 983**

(73) Titulaire: **LABORATOIRE NATIVELLE (Société Anonyme)**
**70, rue du Gouverneur Général Eboué**
**F-92130 Issy les Moulineaux(FR)**

(72) Inventeur: **Jarreau, François-Xavier**
**5, rue Louis Hervé**
**F-78000 Versailles(FR)**
Inventeur: **Koenig, Jean-Jacques**
**31, rue du Panorama**
**F-77670 Vernou La Celle S/Seine(FR)**

(74) Mandataire: **L'Helgoualch, Jean**
**Cabinet Sueur et L'Helgoualch 78, rue Carnot**
**F-95240 Cormeilles-en-Parisis(FR)**

EP 0 310 519 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de stéroïdes, et plus particulièrement de nouveaux esters d'acide androstane 17-carboxylique substitués en position 3 par un reste sucré, un procédé pour leur préparation, leur utilisation pour la fabrication de médicaments, ainsi que les médicaments les contenant.

Le brevet FR 2.464.270 décrit des composés du type amino-14 stéroïdes, et notamment des dérivés hydroxylés de l'amino-14 androstane et de l'amino-14 nor-21 pregnane. On connait également des alcaloïdes stéroïdes stéroïdiques de la série du pregnane et de l'androstane substitués en position 14 par un groupe amino, et par exemple l'amino-14$\beta$ pregnanediol-3$\beta$,20$\alpha$ est décrit par A. Astier et al. Bull. Soc. Chim. n° 9-10 p.1581-1582 (1976); d'autres amino-14$\beta$ pregnanes et amino-14$\beta$ androstanes sont également décrits par A. Astier et al. Tetrahedron vol.34 p.1481-1486 (1978). Toutefois, ces documents ne décrivent aucune propriété pharmacologique ni aucune application thérapeutique possible de ces dérivés.

Les brevets FR 2.531.964 et 2.547.586 décrivent des dérivés du pregnanol-20, du nor-21 pregnanol-20, du pregnanediol-12,20 et du nor-21 pregnanediol-12,20, comportant un groupe amino en position 14 et un reste sucré en position 3. Ces dérivés présentent des propriétés inotropes positives permettant d'envisager leur utilisation comme principes actifs de médicaments pour le traitement de l'insuffisance cardiaque.

Le brevet DD 138.983 décrit des dérivés de cardénolides, caractérisés par la présence d'un groupe lactone en position 17, et comportant un groupe amino en position 14.

On connait par ailleurs des dérivés aminés de stéroïdes utiles en thérapeutique, et par exemple les brevets français 2.494.697 et 2.494.698 décrivent des amino-3 (5$\alpha$) pregnanediol-17$\alpha$, 20, des amino-3 (5$\alpha$) nor-19 pregnanol-20, et des dérivés aminés, présentés comme possédant des propriétés immunothérapeutiques permettant leur application à titre de médicaments pour le traitement des maladies autoimmunes résultant d'une déficience en certains lymphocytes.

Les travaux réalisés par la demanderesse ont montré de manière inattendue que certains esters d'acide androstane 17-carboxylique comportant une fonction ester d'alkyle en position 17 et un reste sucré en position 3, présentent un pouvoir cardiotonique nettement plus élevé et plus stable que celui des produits connus de structure dérivée des stéroïdes ou des cardénolides, tandis que les effets secondaires cardiaques sont atténués.

La présente invention a donc pour objet de nouveaux esters d'acide androstane 17-carboxylique substitués en position 3 par un reste sucré présentant des propriétés cardiotoniques.

L'invention a également pour objet un procédé de préparation des esters d'acide androstane 17-carboxylique substitués en position 3 par un reste sucré, à partir d'un ester d'acide azido-14 étianique.

L'invention concerne également l'application des esters d'acide androstane 17-carboxylique substitués en position 3 par un reste sucré à titre de médicaments cardiotoniques pour le traitement de l'insuffisance cardiaque, les compositions pharmaceutiques renfermant comme principe actif l'un au moins des nouveaux esters d'acide androstane 17-carboxylique substitués en position 3 par un reste sucré ainsi que l'utilisation de ces dérivés pour la fabrication de médicaments utilisables en thérapeutique comme agents cardiotoniques pour le traitement de l'insuffisance cardiaque.

Les nouveaux esters d'acide androstane 17-carboxylique substitués en position 3 par un reste sucré conformes à la présente invention peuvent être représentés par la formule générale (I) suivante:

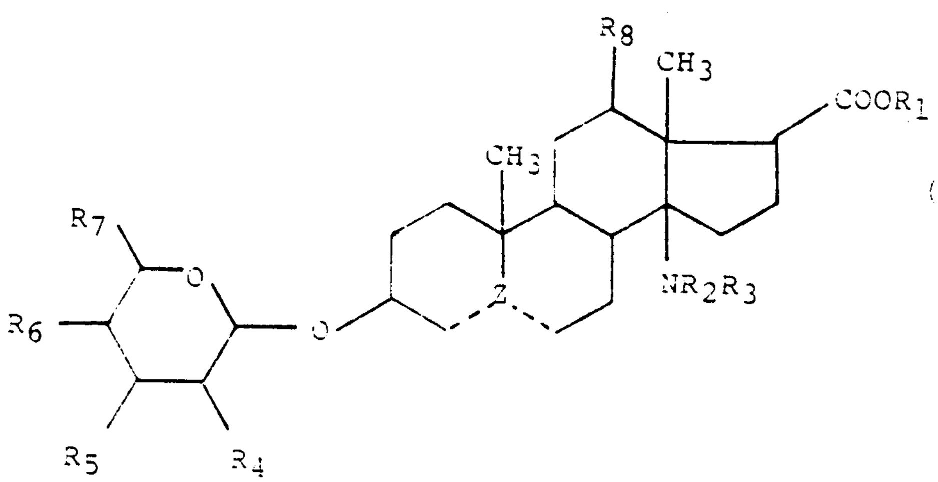

(I)

dans laquelle $R_1$ représente un groupe alkyle inférieur comportant 1 à 4 atomes de carbone, un groupe alkyle inférieur de 2 à 4 atomes de carbone substitué par un groupe amino, ou un groupe aralkyle de 6 à 12 atomes de carbone, $R_2$ et $R_3$, identiques ou différents, représentent un atome d'hydrogène ou un groupe alkyle inférieur comportant 1 à 4 atomes de carbone, $R_4$ représente un atome d'hydrogène, un groupe hydroxy, ou un groupe acétoxy, $R_5$ représente un groupe hydroxy ou acétoxy, ou $R_4$ et $R_5$ représentent ensemble un groupe divalent alkylènedioxy, $R_6$ représente un groupe hydroxy, un groupe méthoxy ou un groupe acétoxy, $R_7$ représente un groupe méthyle ou un groupe hydroxy-méthyle, $R_8$ représente un atome d'hydrogène, un groupe hydroxy ou un groupe acétoxy, et --Z-- représente soit un groupe -CH-, soit un carbone =C- dont la double liaison est en position 4 ou 5 du noyau stéroïde.

Dans la formule générale (I) ci-dessus, le noyau stéroïde peut être saturé comme le montre la formule (Ia) ci-dessous, ou comporter une double liaison en 4-5 ou en 5-6, constituant ainsi un dérivé de type androstène-4 ou -5 représenté sur les formules (Ib) et (Ic) ci-dessous.

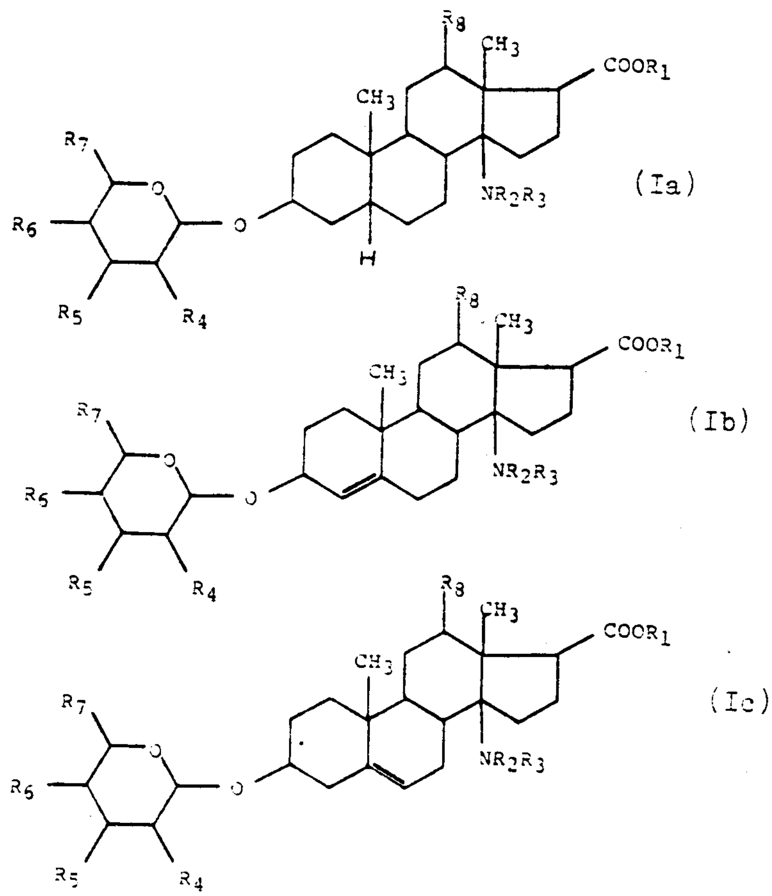

(Ia)

(Ib)

(Ic)

Dans ce qui suit, l'expression "formule générale (I)" désigne globalement les trois variantes (Ia), (Ib) et (Ic), sauf indication contraire.

Dans la formule générale (I) ci-dessus, le groupe alkyle inférieur représenté par $R_1$ peut être un groupe méthyle, éthyle, isopropyle, butyle, etc, et de préférence un groupe méthyle ou éthyle. Le groupe alkyle inférieur ci-dessus peut être substitué par un groupe amino pour former par exemple un groupe amino-2 éthyle ou amino-3 propyle, ou di-(ou mono-) méthylamino-3 propyle. Lorsque $R_1$ représente un groupe aralkyle, ce groupe peut être par exemple un groupe phényle, un groupe benzyle, un groupe tolyle, ou un groupe phénéthyle. $R_2$ et $R_3$, qui peuvent être identiques ou différents représentent de préférence un atome d'hydrogène ou un groupe méthyle. Lorsque $R_4$ et $R_5$ se combinent pour former un groupe divalent alkylènedioxy, ce groupe peut comporter une partie alkylène linéaire ou ramifiée contenant 1 à 4 atomes de carbone, et constituer par exemple un groupe méthylènedioxy, un groupe éthylènedioxy, ou un groupe isopropylidènedioxy. $R_8$ représente de préférence un atome d'hydrogène ou un groupe hydroxy.

Les nouveaux esters d'acide androstane 17-carboxylique conformes à la présente invention comportent dans leur molécule plusieurs atomes de carbone asymétriques et peuvent donc exister sous plusieurs formes stéréoisomères. L'invention concerne bien entendu les nouveaux dérivés représentés par la formule

générale (I) ci-dessus sous formes d'isomères séparés ou en mélange de deux ou plusieurs isomères.

Plus particulièrement, le groupe amino en position 14, le groupe ester en position 17 et le reste sucré en position 3, peuvent avoir la configuration $\alpha$ ou $\beta$, mais ils ont de préférence la configuration $\beta$. Il a été en effet constaté que l'activité pharmacologique des dérivés de formule générale (I) était généralement supérieure lorsque l'un au moins de ces trois substituants, et de préférence le groupe amino en position 14, ont la configuration $\beta$, et plus particulièrement lorsque les trois substituants ont la configuration $\beta$.

L'atome d'hydrogène en position 5 du noyau stéroïde peut avoir l'une ou l'autre des configurations $\alpha$ et $\beta$, mais il a de préférence la configuration $\beta$.

Parmi les nouveaux esters d'acide androstane 17-carboxylique représentés par la formule générale (I) ci-dessus, l'invention concerne de préférence l'$\alpha$-L-rhamnopyranosyloxy-3$\beta$ amino-14$\beta$ étianate de méthyle, l'$\alpha$-L-rhamnopyranosyloxy-3$\beta$ méthylamino-14$\beta$ étianate de méthyle, et l'$\alpha$-L-rhamnopyranosyloxy-3$\beta$ amino-14$\beta$ etièn-4 ate de méthyle.

Comme indiqué ci-dessus, les dérives de formule générale (I) conformes à la présente invention peuvent être préparés à partir d'un ester d'acide 3-hydroxy 14-azido androstane 17-carboxylique, représenté par la formule générale (II) ci-dessous:

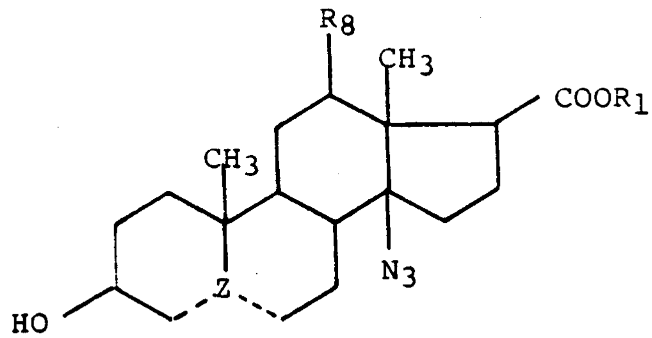

(II)

dans laquelle $R_1$, $R_8$ et Z ont la même signification que dans la formule générale (I), que l'on fait réagir avec un dérivé activé de sucre par réaction de couplage, pour former un ester d'acide 3-pyranosyloxy 14-azido androstane 17-carboxylique de formule générale (III) ci-après:

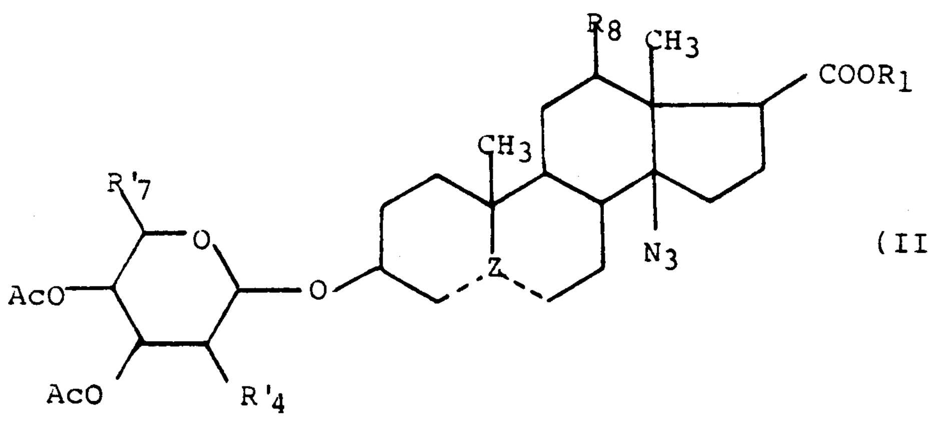

(III)

dans laquelle $R_4'$ représente un atome d'hydrogène ou un groupe acétoxy, $R'_7$ représente un groupe méthyle ou un groupe $-CH_2OAc$, $R_1$, $R_8$ et Z possèdent la même signification que dans la formule générale (I), que l'on réduit en dérivé de formule générale (I), puis on effectue si nécessaire une aminométhylation.

Les dérivés de formule générale (I) dans laquelle $R_2$ et $R_3$ représentent tous deux un atome d'hydrogène, comportant donc un groupe amino en position 14, peuvent être transformés en dérivés comportant un group mono- ou di-alkylamino par réaction d'aminométhylation suivant les techniques usuelles.

L'ester d'acide 3-hydroxy 14-azido androstane 17-carboxylique de formule générale (II) utilisé comme produit de départ peut être aisément obtenu à partir de l'acétoxy-3 azido-14 étianate de méthyle décrit dans le brevet FR 2.464.270, par une réaction de désacétylation usuelle, par exemple par action d'un

alcoolate de métal alcalin tel que le méthanolate de sodium, dans un solvant approprié.

L'ester d'acide 3-hydroxy 14-azido androstane 17-carboxylique de formule générale (II) comportant une double liaison en 4-5 ou en 5-6 (Z est $=\overset{|}{C}-$ ou $-\overset{|}{C}=$) peut être aisément préparé à partir de l'ester correspondant comportant un noyau stéroïde saturé, par les techniques usuelles de formation d'une double liaison, par exemple comme décrit par J. Fried et J.A. Edwards Organic Reaction in Steroid Chemistry Van Nostrand Rheinhold Co. (1971) et dans les exemples ci-après.

Le dérivé de sucre, utilisé dans la réaction de couplage avec l'ester d'acide 3-hydroxy 14-azido androstane 17-carboxylique, est un dérivé activé. L'activation peut être réalisée par exemple en transformant le sucre en un dérivé halogéné ou acétylé. Le dérivé halogéné est de préférence un bromure, par exemple le bromure de rhamnosyle que l'on fait agir en présence de cyanure mercurique. Dans le cas d'une activation par acétylation, on peut utiliser par exemple un tri-O-acétyl digitoxose que l'on fait réagir avec l'ester d'acide 3-hydroxy 14-azido androstane 17-carboxylique en présence d'acide toluène sulfonique.

D'une manière générale, le sucre activé, et protégé comme indiqué ci-dessous, peut être un monosaccharide tel qu'un hexose, un désoxy-2 hexose, un desoxy-6 hexose, un didésoxy-2,6 hexose, etc. Plus particulièrement, le monosaccharide peut être choisi parmi un rhamnose, un glucose, un arabinose, un digitoxose, un fructose, un galactose; on peut utiliser par exemple un rhamnopyrannose, un hexopyrannose, le désoxy-6 glucose, le didésoxy-4,6 glycopyrannose, etc. On utilise de préférence un rhamnose, et plus particulièrement un rhamnopyrannose. On peut bien entendu utiliser les divers anomères existants, et de préférence les formes $\alpha$-L et $\beta$-D.

Les fonctions hydroxyle du sucre sont préalablement protégées de manière usuelle avant de réaliser la réaction de couplage, par exemple par acétylation ou par benzoylation. Une technique de protection des sucres utilisable dans la présente invention est décrite par exemple par E. Fisher et al. Chem. Ber. 53, p.2362 (1920). Le dérivé activé de sucre ainsi protégé peut être par exemple le tri-O-acétyl-2,3,4 bromo-1 rhamnose que l'on fait réagir en présence de cyanure mercurique, ou le tri-O-acétyl digitoxose en présence d'acide toluène sulfonique; dans ce deuxième cas, on obtient un mélange de glycosides isomères que l'on peut séparer par chromatographie après désacétylation. Lorsque $R_8$ représente un groupe hydroxyle, ce groupe est également de préférence préalablement protégé par les mêmes techniques.

Les groupements protecteurs des dérivés sucrés peuvent être ensuite éliminés par les techniques usuelles, après le couplage avec l'ester d'acide azido-14 étianique, si on le désire. La déprotection peut s'effectuer par exemple par chaufage en milieu basique.

Comme indiqué ci-dessus, après la réaction de couplage avec le dérivé sucré, on effectue une réduction pour obtenir le dérivé de formule générale (I). La réduction du pyranosyloxy-3 azido-14 étianate de méthyle de formule générale (III) est réalisée par action du borohydrure de sodium en présence de tellure dans un alcool, suivie si nécessaire d'une désacétylation.

Suivant une variante, la réduction du pyranosyloxy-3 azido-14 étianate de méthyle de formule générale (III) est réalisée par réaction d'hydrogénation en présence de nickel Raney, précédée si nécessaire d'une désacétylation.

Les exemples suivants illustrent l'invention plus en détail sans en limiter la portée.

**EXEMPLE 1**

$\alpha$-L-rhamnopyranosyloxy-3$\beta$ amino-14$\beta$ étianate de méthyle

On prépare une solution contenant 20g d'hydroxy-3$\beta$ azido-14$\beta$ étianate de méthyle et 37,6g de tri-O-acétyl-2,3,4 bromo-1 rhamnose dans 1,5 litre d'acétonitrile distillé extemporanément sur $P_2O_5$. On ajoute 26,7g de cyanure mercurique, et on agite à température ambiante pendant environ une heure.

Le milieu réactionnel est ensuite versé dabs 650ml d'une solution saturée d'hydrogénocarbonate de sodium. Cette solution est agitée pendant 50 min, puis additionnée de 500ml de toluène; la phase aqueuse est extraite trois fois au toluène, lavée à l'eau, puis à l'eau saturée de chlorure de sodium.

Les phases toluéniques sont réunies, séchées, concentrées à sec; après recristallisation dans le méthanol, on obtient ainsi 26,2g (rendement 76%) de cristaux blancs de (tri-O-acétyl $\alpha$-L-rhamnopyranosyloxy)-3$\beta$ azido-14$\beta$ étianate de méthyle.

L'hydroxy-3$\beta$ azido-14$\beta$ étianate de méthyle utilisé comme produit de départ est obtenu à partir du dérivé acétoxylé correspondant, par chauffage en présence de méthanolate de sodium, extraction et cristallisation dans l'hexane.

Dans un ballon d'un litre, on porte à reflux pendant environ 6 heures sous argon, un mélange de 8,7g de borohydrure de sodium et de 12,3g de tellure pulvérisé, dans 313ml d'éthanol absolu distillé extemporanément sur magnésium et dégazé à l'azote.

Après refroidissement à température ambiante, on ajoute progressivement, sous atmosphère inerte, 25g de (tri-O-acétyl α-L-rhamnopyranosyloxy)-3β azido-14β étianate de méthyle obtenu comme indiqué ci-dessus, et on maintient sous agitation à température ambiante pendant 20 heures environ.

Le mélange réactionnel est ensuite agité à l'air pendent 30 minutes, filtré sur célite, rincé à l'éthanol absolu, et concentré à sec. Le solide blanc obtenu est repris par 330ml d'acétate d'éthyle, puis on effectue une extraction acide-base (extraction par l'acide sulfamique à 5%, lavage par l'acétate d'éthyle, alcalinisation agitation pendant une heure, extraction au chlorure de méthylène, puis lavage à l'eau), d'où l'on on obtient 14,3g de bases sous forme de poudre blanche (rendement brut: 75%).

Le produit obtenu, purifié par chromatographie sur silice Merck (solvant: $CH_2Cl_2$/MeOH/$NH_4OH$ 85/15/1,5 Rf = 0,32) puis recristallisation dans l'éthanol, est l'α-L-rhamnopyranosyloxy-3β amino-14β étianate de méthyle.

Point de fusion: F = 253/254 ° C (solvant EtOH/$H_2O$)

Spectre IR (Nujol): ν = 3370, 3320, 1735, 1605 $cm^{-1}$

Spectre de RMN: ($CDCl_3$ + $CD_3OD$ 3,5/1) δ = 0,91 (s, Me-19) 0,93 (s, Me-18) 1,27 (d, Me-6', $J_{5'-6'}$ = 6Hz) 3,68 (s, -$COOCH_3$) 4,80 (H-1') ppm.

**EXEMPLE 2**

α-L-rhamnopyranosyloxy-3β méthylamino-14β étianate de méthyle

On dissout 0,16g du dérivé obtenu comme indiqué dans l'exemple 1 dans 3,5ml d'acétonitrile, on ajoute 0,03ml de formaldéhyde en solution aqueuse à 35%, et 22mg de borocyanohydrure de sodium. On laisse la réaction se développer pendant 15 minutes.

Après neutralisation par l'acide acétique et évaporation du solvant, reprise par de la potasse 2N et extraction des bases, on obtient 155mg de produit de méthylation que l'on sépare par chromatographie sur colonne de silice.

Chromatographie CCM: Rf = 0,3

Eluant: $CH_2Cl_2$/MeOH/$NH_4OH$ (80/20/2)

Spectre IR (Nujol): ν = 3700-2300 (max. 3360), 1725, 1695 $cm^{-1}$.

**EXEMPLE 3**

α-L-rhamnopyranosyloxy-3β diméthylamino-14β étianate de méthyle

Ce dérivé est formé simultanément avec celui de l'exemple 2 ci-dessus. Il est isolé par chromatographie sur colonne et cristallisation dans l'éthanol.

Chromatographie CCM: Rf = 0,4

Eluant: $CH_2Cl_2$/MeOH/$NH_4OH$ (85/15/1,5)

Spectre IR (Nujol) ν = 3620-2500 (max. 3550, 3380), 2780, 1735, 1705 $cm^{-1}$.

**EXEMPLE 4**

α-L-rhamnopyranosyloxy-3β amino-14β étianate d'éthyle

On fait réagir 0,3g du dérivé obtenu comme indiqué dans l'exemple 1 dans 3,5ml d'éthanol contenant un équivalent d'éthanolate de sodium préparé extemporanément.

On laisse réagir pendant 24 heures. Après extraction et lavage suivant la même technique que dans l'exemple 1, puis trituration dans l'éther isopropylique et cristallisation dans l'éther éthylique, on obtient 0,2g de produit pur constitué par l'α-L-rhamnopyranosyloxy-3β amino-14β étianate de méthyle.

Chromatographie CCM: Rf = 0,25

Eluant: $CH_2Cl_2$/MeOH/$NH_4OH$ (85/15/15)

Spectre IR (Nujol) ν = 3250-3600, 1730, 1700, 1660, 1515, 1040$cm^{-1}$.

**EXEMPLE 5**

(O-méthyl-4' α-L-rhamnopyranosyloxy)-3β amino-14β étian-5β,17α(H)-ate de méthyle

On procède comme indiqué dans l'exemple 1 à partir d'hydroxy-3β azido-14β étianate de méthyle mais

en remplaçant le tri-O-acétyl-2,3,4 bromo-1 rhamnose par le di-O-acétyl-2,3 méthoxy-4 bromo-1 rhamnose dans 1,5 litre d'acétonitrile distillé.

On obtient ainsi le (O-méthyl-4' α-L-rhamnopyranosyloxy)-3β azido-14β étian-5β,17α(H)-ate de méthyle, dont on place 70 mg en suspension dans 1,5ml de mélange constitué par 40mg de tellure, 28mg de borocyanohydrure de sodium et 1,5ml d'éthanol absolu. Le mélange réactionnel est maintenu sous agitation à température ambiante pendant 21 heures environ sous atmosphère d'argon.

L'excès de réactif est détruit par agitation pendant 15 minutes à l'air libre, puis le mélange réactionnel est filtré sur célite en rinçant par un mélange de chlorure de méthylène et d'éthanol. Après évaporation du filtrat à sec, extraction par le toluène, lavage à l'acide sulfamique en solution aqueuse à 2%, alcalinisation et extraction au chlorure de méthylène, on obtient 57mg de base (rendement 85%).

On effectue une purification par chromatographie sur colonne de silice sous pression en utilisant comme éluant le mélange $CH_2Cl_2/EtOH/N_4OH$ (89/1/9/0,9). Après cristallisation dans l'isopropanol, on obtient 47mg d' (O-méthyl-4' α-L-rhamnopyranosyloxy)-3β amino-14β étian-5β,17α(H)-ate de méthyle (rendement 71%).

Point de fusion: F = 106-108 ° C (solvant isopropanol)

| | |
|---|---|
| Spectre IR (Nujol) ν = | 3700-3100 (max. 3360), 1725, 1600, 1195, 1170, 1110, 1045, $cm^{-1}$ |
| Spectre de RMN ($CDCl_3/CD_3OD$) δ = | 0,90 (s, Me 19), 0,94 (s, Me 18), 1,26 (d, J=6, Me 6'), 2,51 (m, H 17) 3,02 (t,J=9,5, H 4'), 3,54 (s, C-OMe), 3,65 (s, COOMe), 3,4 à 4,1 (m, H 2', 3' 5' et H 3), 4,75 (s, H 1') ppm. |

**EXEMPLE 6**

(désoxy-6' α-L-tallo-hexopyranosyloxy)-3β amino-14β étian-5β,17α(H)-ate de méthyle

En opérant sous atmosphère d'azote, on ajoute 1,8g de (tri-O-acétyl α-L-rhamnopyranosyloxy)-3β azido-14β étianate de méthyle, obtenu comme indiqué dans l'exemple 1, à 8,4ml de méthylate de sodium 1N. on ajoute ensuite 2,5ml de méthanol et on laisse réagir pendant environ 1 heure à température ambiante.

Après neutralisation par l'acide acétique glacial, et extraction par le chlorure de méthylène, on obtient 1,4g d'α-L-rhamnopyranosyloxy-3β azido-14β étianate de méthyle. On en prélève 0,5g que l'on dissout dans 4ml de diméthylformamide, puis on ajoute 5ml de diméthoxy-2,2 propane et 15ml d'acide tosylique. On laisse réagir pendant 2 heures à température ambiante, et après évaporation du solvant et extraction au toluène, on obtient 0,4g (rendement brut 96%) de (O-isopropylidène-2',3' α-L-rhamnopyranosyloxy)-3β azido-14β étian-5β,17α(H) ate de méthyle.

Le dérivé ainsi obtenu est purifié par chromatographie sur colonne de silice sous pression, en éluant par le mélange $CH_2Cl_2/EtOH$ (98/2) (Rf = 0,65).

Spectre IR (Nujol) ν = 3650-3250 (max. 3470),2110, 1740 $cm^{-1}$.

A une solution refroidie à -60 ° C de 0,1ml de chlorure d'oxalyle dans 2,1ml de chlorure de méthylène, on ajoute 0,1ml de diméthylsulfoxyde sec. On maintient sous agitation pendant 5 minutes à -60 ° C, puis pendant 5 minutes à -40 ° C, et on ajoute goutte à goutte 100mg d'(O-isopropylidène-2',3' α-L-rhamnopyranosyloxy)-3β azido-14β étian-5β,17α(H)-ate de méthyle obtenu comme indiqué ci-dessus, dissous dans 0,7ml de chlorure de méthylène.

Le mélange réactionnel est maintenu sous agitation pendant une heure à -40 ° C, puis on arrête la réaction en ajoutant 0,6ml de N,N-diisopropyléthylamine.

On laisse le milieu revenir à température ambiante et on évapore le solvant sous vide, pour obtenir le (désoxy-6' O-isopropylidene-2',3'α-L-lyxohexopyranulos-4'-yloxy)-3β azido-14β étian-5β,17α(H)-ate de méthyle.

A une solution refroidie à 0 ° C de 100mg du dérivé obtenu comme indiqué ci-dessus, et de 66mg de $CeCl_3$ $7H_2O$ dans 0,5ml de méthanol, on ajoute 6,7g de borohydrure de sodium. On obtient ainsi une supension que l'on maintient sous agitation à température ambiante pendant une heure. Après extraction au chlorure de méthylène et lavage à l'eau saturée de chlorure de sodium, le résidu est purifié par chromatographie sur colonne de silice sous pression en éluant par le mélange $CH_2Cl_2/EtOH$ (98/2) (Rf = 0,46).

Le (désoxy-6' O-isopropylidène-2',3' α-L-tallo-hexopyranosyloxy)-3β azido-14β étian-5β,17α(H)-ate de méthyle ainsi obtenu (70mg), est hydrolysé en solution dans 1,4ml de chloroforme par 0,16ml d'acide trifluoroacétique en présence d'environ 1% d'eau. Après extraction par le chloroforme, on obtient 70mg de résidu sec cristallisable dans le méthanol, constitué par le (désoxy-6' α-L-tallohexopyraosyloxy)-3β azido-14β étian-5β,17α(H)-ate de méthyle.

On réduit le dérivé ainsi obtenu par un mélange de borohydrure de sodium (26mg), de tellure pulvérisé (37mg) et d'éthanol absolu (2ml) suivant la méthode décrite dans l'exemple 1, en laissant la réaction s'effectuer pendant une nuit.

Après filtration, rinçage, extraction, comme indiqué dans l'example 1, on obtient le (désoxy-6' α-L-tallo-hexopyranosyloxy)-3β amino-14β étian-5β,17α(H)-ate de méthyle (isomère du dérivé de l'exemple 1).

Point de fusion: F = 210°C (solvant isopropanol)

| | |
|---|---|
| Spectre IR (Nujol): ν = | 3600-3050 (3480, 3370, 3100) 1725, 1600, 1105, 1100, 1050, 1025, 975 $cm^{-1}$ |
| Spectre de RMN: ($CDCl_3$/$CD_3OD$ 4/1) δ = | 0,92 (s, Me-19) 0,94 (s, Me-18) 1,25 (d, J=6, Me 6') 2,55 (m, H 17), 3,67 (s, $OCH_3$), 4,08 (s, H 1') 7,44 ($CHCl_3$) ppm |

**EXEMPLE 7**

(O-isopropylidène-2', 3' α-L-rhamnopyranosyloxy)-3β amino-14β étian-5β,17α(H)-ate de méthyle

A 5,4ml d'éthanol absolu désoxygéné par de l'argon, on ajoute 150mg de tellure pulvérisé et 103mg de borohydrure de sodium. On chauffe pendant environ 2 heures à reflux de l'ethanol, sous argon. Après refroidissement à température ambiante, on ajoute 270mg d'(O-isopropylidène-2',3' α-L-rhamnopyranosyloxy)-3β azido-14β étian-5β,17α(H) ate de méthyle obtenu comme indiqué dans l'exemple 6, en solution dans 2ml d'éthanol absolu désoxygéné.

Le milieu réactionnel est maintenu sous agitation pendant 21 heures, puis la réaction est arrêtée en maintenant 15 minutes à l'air libre pour détruire l'excès de réactif.

On filtre sur colonne de célite en éluant par le mélange $CH_2/Cl_2$/EtOH (9/1), puis on évapore le filtrat à sec. Le résidu est repris par du toluène puis on extrait les bases à l'acide sulfamique en solution aqueuse à 2%. Après alcalinisation par le carbonate de sodium et extraction par le chlorure de méthylène, on obtient 189mg de base et 42mg de neutre.

En saturant les phase aqueuses par du chlorure de sodium, on extrait à nouveau 26mg de produit très polaire également présent dans le premier extrait basique.

La fraction basique principale est purifiée par chromatographie sur colonne de 5,7g de silice sous 500mB de pression, an éluant par le mélange $CH_2Cl_2$/EtOH/$NH_4OH$ (94,5/5/05).

On obtient ainsi 150mg de (O-isopropylidène-2',3', α-L-rhamnopyranosyloxy)-3β amino-14β étian-5β,17α(H)-ate de méthyle rendement 58%) sous forme de cristaux incolures solubles dans l'ether isopropylique.

Point de fusion: F = 179°C (solvant éther isopropylique)

| | |
|---|---|
| Spectre IR (Nujol): ν = | 3700-3050 (max. 3350, 3300, 3170) 1735, 1600, 1170, 1075, 1555 $cm^{-1}$ |
| Spectre de RMN: ($CDCl_3$) δ = | 0,91 (s, Me-19) 0,94 (s, Me-18) 1,25 (d, J=6, Me 6') 1,36 et 1,51 (2s, $CH_3$-C-$CH_3$) 2,51 (m, H 17), 3,64 (s, $OCH_3$), 3,2 à 4,2 (m, H 2', 3', 4', 5', H 3) 4,99 (s, H 1') ppm. |

**EXEMPLE 8**

O-(didésoxy-2',6' α-D-ribo-hexopyranosyloxy)-3β amino-14β étian-5β-ate de méthyle

On maintient sous agitation, pendant 2 heures, à température ambiante, une solution de 1,6g d'hydroxy-3β azido-14β étianate de méthyle et de 2,3g de tri-O-acétyl digitoxose en présence de 1,9g d'acide p-toluène sulfonique sec, dans 24ml de benzène.

Après addition d'une solution aqueuse saturés de bicarbonate de sodium, et extraction par le benzène, on obtient un résidu qui est purifié par chromatographie sur colonne de silice sous pression, en éluant par le mélange AcOEt/hexane (25/75). On obtient ainsi un mélange de glycosides acétylés, que l'on soumet à une réaction de désacétylation par 5,3ml de méthylate de sodium. Les produits sont ensuite séparés par chromatographie sur colonne.

L'α-D-digitoxoside séparé (CCM: éluant $CH_2Cl_2$MeOH 95/5, Rf = 0,57) est utilisé tel quel dans l'étape de réduction suivante.

La réduction s'effectue suivant la technique décrite dans l'exemple 1, par le mélange de borohydrure de sodium, de tellure pulvérisé et d'éthanol.

Après extraction comme indiqué dans l'exemple 1, et chromatographie sur colonne de silice sous pression, en éluant par le mélange $CHCl_3$/EtOH/$NH_4OH$ (94,5/5/0,5), et cristallisation dans le mélange

acétate d'éthyle - éther isopropylique, on obtient des cristaux d'O-(didésoxy-2',6' α-D-ribo-hexopyranosyloxy)-3β amino-14β étian-5β-ate de méthyle.
Point de fusion: F = 178-182 °C (solvant acétate d'éthyle - éther isopropylique)
Spectre IR (Nujol): ν = 3520, 3470, 1730 $cm^{-1}$
Spectre de RMN: ($CDCl_3$/$CD_3OD$ 4/1)) δ = 0,94(s, Me-18 et Me-19) 1,31 (d, J=6, Me 6') 2,52 (m, H-17), 3,11 (dd, $J_{3'4'}$=3, $J_{4'5'}$=10, H-4') 3,66 (s, $OCH_3$), 3,95 (H-3, H-3') 4,95 (s, H 1') 7,37 ($CHCl_3$) ppm.

**EXEMPLE 9**

O-(didésoxy-2',6' β-D-ribo-hexopyranosyloxy)-3β amino-14β étian-5β-ate de méthyle

On procède comme indiqué dans l'exemple 8 et on sépare le β-D-digitoxoside, isomère de l'α-D-digitoxoside de l'exemple 8, par chromatographie.

Le β-D-digitoxoside ainsi obtenu est réduit suivant la technique de l'exemple 8 par le mélange de borohydrure de sodium, de tellure pulvérisé et d'éthanol).

Après extraction des bases comme indiqué dans l'exemple 1, chromatographie sur colonne de silice sous pression en éluant par le mélange $CHCl_3$/EtOH/$NH_4OH$ (92,3/7/0,7), et cristallisation dans le mélange acétate d'éthyle - éther isopropylique, on obtient le O-(didésoxy-2',6' β-D-ribo-hexopyranosyloxy)-3β amino-14β étian-5β-ate de méthyle.
Point de fusion: F = 162-167 °C (solvent acétate d'éthyle - éther isopropylique)
Spectre IR (Nujol): ν = 3600-3040 (3350), 1725, 1595, 1070$cm^{-1}$
Spectre de RMN: ($CDCl_3$/$CD_3OD$ 4/1)) δ = 0,95 et 0,96 2(s, Me-18 et Me-19) 1,27 (D, J=6, Me 6') 2,54 (m, H-17), 3,21 (dd, $J_{3'4'}$=3, $J_{4'5'}$=10, H-4') 3,70 (s, $OCH_3$), 4,05 (H-3, H-3') 4,90 (dd, $J_{1'2'a}$=10, $J_{1'2'e}$=2, H 1') 7,52 ($CHCl_3$) ppm.

**EXEMPLE 10**

O-(didésoxy-2',6' β-D-arabino-hexopyranosyloxy)-3β amino-14β étian-5β,17α(H)-ate de méthyle

On effectue un couplage de tri-O-acétyl digitoxoside et d'hydroxy-3β azido-14β étianate de méthyle suivant la technique décrite dans l'exemple 8, puis on effectue une saponification par le méthylate de sodium, et une chromatographie sur colonne de silice sous pression suivant la technique usuelle.

On obtient ainsi le O-(didésoxy-2',6' β-D-arabinohexopyranosyloxy)-3β azido-14β étian-5β,17α(H) ate de méthyle, sur lequel on effectue une réaction de réduction par la technique indiquée ci-dessus.

Après extraction et chromatographie sur colonne de silice sous pression, en éluant par le mélange $CH_2Cl_2$/EtOH/$NH_4OH$ (89/10/1), on obtient le O-(didésoxy-2'6' β-D-arabino-hexopyranosyloxy)-3β amino-14β étian-5β,17α(H)-ate de méthyle.
Point de fusion: F = 210 °C (solvant: isopropanol - éther isopropylique)
Spectre IR (film $CHCl_3$): ν = 3700-3100 (3360, 3220), 1700, 1625, 1215 $cm^{-1}$
Spectre de RMN: ($CDCl_3$/$CD_3OD$ 4/1)) δ = 0,93 2(s, Me-19), 1,01 (s, Me-18) 1,29 (d, J=6, Me 6'), 3,72 (s, $0CH_3$), 4,03 (H-3) 4,56 (dd, J=9, H-1') 7,35 ($CHCl_3$) ppm.

**EXEMPLE 11**

O-(didésoxy-2',6' α-D-arabino-hexopyranosyloxy)-3β amino-14β étian-5β,17α(H)-ate de méthyle

On procède comme dans l'exemple 10, mais en utilisant comme intermédiaire le O-(didésoxy-2'6' β-D-arabinohexo-pyranosyloxy)-3β azido-14β étian-5β,17α(H) ate de méthyle, isomère β-D du glycoside α-D de l'exemple 10, isolé par chromatographie à partir du même mélange réactionnel.

La réduction dans les mêmes conditions que dans l'exemple 10 fournit, après chromatographie sur colonne de silice sous pression, en éluant par le mélange $CH_2Cl_2$/EtOH/$NH_4OH$ (89/10/1), et cristallisation dans le mélange $CH_2Cl_2$/EtOH, des cristaux de O-(didésoxy-2',6' α-D-arabino-hexopyranosyloxy)-3β amino-14β étian-5β,17α(H)-ate de méthyle.
Point de fusion: F = 270 °C (solvant: chlorure de méthylène - isopropanol)
Spectre IR (Nujol): ν = 3500-3150 (3350, 3270), 1730,1605$cm^{-1}$

**EXEMPLE 12**

α-L-rhamnopyranosyloxy-3β amino-14β etièn-4-ate de méthyle

On traite 5g d'hydroxy-3β azido-14β étianate de méthyle par 5 ml de réactif de Jones dans 100ml d'acétone. Le réactif est ajouté goutte à goutte dans la solution refroidie sur bain de glace. on laisse la réaction d'oxydation s'effectuer pendant 20 minutes, et, après extraction, on obtient 5g de cétone qui cirstallise dans l'acétate d'éthyle (F = 189-191 ° C).

On effectue une bromation de la cétone obtenue comme indiqué ci-dessus en coulant goutte à goutte 1,5g de brome en solution dans 36ml de diméthylformamide dans une solution de 75ml de diméthylformamide contenant 2,2g de cétone, en présence de 0,07g de TsOH. L'addition s'effectue en 30 minutes. On laisse reposer 6 heures à température ambiante, et après extraction on obtient un mélange de deux isomères que l'on sépare par chromatographie sur colonne de silice (acétate d'éthyle/hexane 22/78) pour procurer 1,5g d'oxo-3 bromo-4β azido-14β étian-5β-ate de méthyle (fraction la plus polaire) et 0,5g d'oxo-3 bromo-2β azido-14β étian-5β-ate de méthyle (fraction la moins polaire).

On fait réagir 0,8 d'oxo-3 bromo-4β azido-14β étian-5β-ate de méthyle dans 20ml de diméthylformamide avec 0,7g de chlorure de lithium, en chauffant pendant 2 heures à 110 ° C sous azote. Après extraction et chromatographie sur silice (acétate d'éthyle/hexane 28/72) on obtient 0,5g d'oxo-3 azido-14β étièn-4-ate de méthyle (chromatographie sur couche mince: acétate d'éthyle/hexane 1/1, Rf = 0,3)

La cétone obtenue comme indiquée ci-dessus est réduite en faisant agir 0,5g de $CeCl_3$ puis 50mg de $NaBH_4$ sur 0,5g de cétone dans 10ml de méthanol. On acidifie par de l'acide chlorhydrique N (pH 6), et on extrait par l'acétate d'éthyle pour obtenir 0,5g d'hydroxy-3β azido-14β étièn-4-ate de méthyle.
Chromatographie sur couche mince: $CH_2Cl_2$/MeOH 97/3, Rf = 0,35)

Spectre IR (Nujol) ν = 3000-3600 (3520, 3340) 2100, 1715, 1665, 1285, 1205, 1180 $cm^{-1}$.

L'hydroxy-3β azido-14β étièn-4-ate de méthyle ainsi obtenu, analogue du produit de départ de l'exemple 1 comportant une double liaison en position 4, est traité comme dans l'exemple 1 par le tri-O-acétyl-2,3,4 bromo-1 rhamnose et le cyanure mercurique pour former le (tri-O-acétyl α-L-rhamnopyranosyloxy)-3β azido-14β étièn-4-ate de méthyle.

On effectue une désacétylation du (tri-O-acétyl α-L-rhamnopyranosyloxy)-3β azido-14β étianate de méthyle par action du méthanolate de sodium dans le méthanol à température ambiante, puis neutralisation par l'acide chlorhydrique dilué et extraction par $CH_2Cl_2$.

Le dérivé azido-14 ci-dessus est réduit en dérivé amino-14 correspondant par action du borohydrure de sodium en présence de tellure pulvérisé, dans l'éthanol absolu, suivant le même procédé que dans l'exemple 1.

Après chromatographie sur silice ($CH_2Cl_2$/MeOH/$NH_4OH$ 85/12/06) on obtient l'α-L-rhamnopyranosyloxy-3β amino-14β étianate de méthyle recherché qui cristallise dans l'éthanol absolu.
Point de fusion: F = 240-246 ° C.

Spectre de RMN ($CDCl_3$ + $CD_3O_4$ 4/1) δ = 1,02 (s, Me-18 + Me-19), 1,26 (d, J = 6, Me-6'), 2,6 (m, H-17)
3,69 (s, MeO), 4,86 (s, H-1'), 5,26 (s, H-4) ppm

**EXEMPLE 13**

α-L-rhamnopyranosyloxy-3β amino-14β étian-5α-ate de méthyle

On traite 37mg de l'ester de l'exemple 12 en présence de 20mg de $PtO_2$ selon la réaction d'Adams. On agite le mélange en suspension dans 6ml d'acétate d'éthyle et 0,6ml d'acide acétique pendant 48 heures.

Après traitement d'extraction selon la technique usuelle, à partir de la fraction basique, on obtient 28mg de produit que l'on purifie par cristallisation dans l'éther isopropylique pour obtenir 16mg de α-rhamnopyranosyloxy-3β amino-14β étian-5α-ate de méthyle.
Point de fusion: F = 206 ° C

Spectre de RMN ($CHCl_3$/MeOH 3/1) δ = 0,80-0,98 (s, Me-18 + Me-19), 1,26 (d, J = 6, Me-6'), 2,6 (m, H-17) 3,71 (s, OMe), 4,85 (s, H-1') ppm.

Les études pharmacologiques et cliniques effectuées sur les nouveaux esters d'acide androstane 17-carboxylique selon la présente invention ont montré qu'ils possèdent d'intéressantes propriétés permettant leur application à titre de principes actifs de médicaments utilisables en thérapeutique humaine et vétérinaire.

Les dérivés suivant l'invention présentent en particulier un effet inotrope positif très important, nettement supérieur à celui de la digoxine, médicament de référence bien connu pour le traitement des insuffisances cardiaque. De plus, leur activité est plus stable et leur toxicité cardiaque est plus faible.

L'activité inotrope, qui démontre le pouvoir cardiotonique, a été vérifiée sur l'oreillette gauche stimulée de cobaye, par mesure de la force de contraction, suivant la technique décrite par J. Ghysel-Burton et al. "Brit. J. Pharmacol." 1979, 66, 2, 175-184.

A titre d'exemple, le dérivé de l'exemple 1 suivant la présente invention développe un effet inotrope positif maximum très important (130% à la concentration de $10^{-5}$ M), tandis que l'effet inotrope de la digoxine, dans les mêmes conditions, est nettement inférieur (70% à la concentration de $10^{-6}$ M). De plus, l'effet inotrope positif du dérivé conforme à l'invention est stable pendant environ 60 minutes, tandis que la digoxine induit rapidement un inotropisme négatif. Ce phénomène indique une toxicité cellulaire moindre pour le dérive de l'invention par comparaison avec la digoxine. De même, l'effet contracturant bien connu des digitaliques, et notamment de la digoxine (cf. Handbook of cardiac glycosides, Ed. K, Greef), est nettement inférieur dans le cas des dérivés de l'invention.

L'effet inotrope positif des dérives conformes à la présente invention décrits dans les exemples 1 à 11 ci-dessus est indiqué dans le tableau ci-dessous. L'effet inotrope est exprimé en pourcentage d'augmentation de la force de contraction de l'oreillette gauche stimulée de cobaye, à la concentration de $5.10^{-6}$ M.

| EXEMPLE | 1 | 2 | 3 | 4 | 5 | 6 | 7 | 8 | 9 | 10 | 11 | 12 | 13 |
|---|---|---|---|---|---|---|---|---|---|---|---|---|---|
| Effet inotrope | 97 | 36 | 17 | 27 | 29 | 34 | 37 | 11 | 35 | 29 | 16 | 95 | 41 |

L'effet inotrope positif chez le chien anesthésié au pentobarbital sodique (essais in vivo) est confirmé par la mesure des paramètres hémodynamiques, selon la méthode décrite par R.E. Patterson et al. "Cardiology" 1972, 57, 277-294. On constate que le produit de l'exemple 1, après perfusion par voie intraveineuse de 3 à $4.10^{-9}$ mole par kg.min pendant 20 minutes, est d'environ 20 à 40%. Si la perfusion est prolongée pendant plusieurs heures à la vitesse de $2{,}2.10^{-8}$ mole par kg.min, l'activité cardiotonique se développe et se maintient à environ 60-70%. Dans le cas de la digoxine, pour un effet inotrope comparable, lorsque la perfusion est maintenue, des troubles du rythme cardiaque apparaissent dès la deuxième heure, puis la mort dès la troisième heure.

Des essais comparatifs ont été effectués sur 10 chiens pour les indices thérapeutiques suivants: A - dose d'arythmie réversible / dose efficace; B - dose toxique / dose efficace; C-dose léthale / dose efficace. La dose d'arythmie réversible est la dose à laquelle apparaît une arythmie spontanément réversible; la dose toxique est la dose à laquelle l'arythmie est continue; la dose léthale est la dose provoquant la mort (pression sanguine et débit cardiaque nuls); la dose efficace est la dose à laquelle on observe une augmentation de 30% de la force de contraction cardique par rapport au niveau témoin.

Les résultats sont indiqués au tableau ci-desous.

| Substance | Indice thérapeutique | | |
|---|---|---|---|
| | A | B | C |
| Digoxine | 1,5 | 2,0 | 3,2 |
| Exemple 1 | 3,1 | 5,8 | 14,0 |

Ces résultats montrent que les dérivés suivant la présente invention possèdent une efficacité supérieure à celle de la digoxine avec une mondre toxicité.

Les esters d'acide androstane 17-carboxylique suivant la présente invention peuvent être utilisés comme principes actifs de médicaments, que l'on peut administrer sous les formes usuelles, le principe actif étant dilué dans un excipient pharmaceutiquement acceptable convenablement choisi, par exemple sous forme de comprimé, gélule, ou soluté injectable.

Les doses administrées sont comparables à celles des médicaments cardiotoniques connus dérivés des cardénolides, et sont déterminées par le médecin de manière usuelle en fonction du mode d'administration et de l'état du patient.

## Revendications

**1.** Esters d'acide androstane 17-carboxylique, représentés par la formule générale (I):

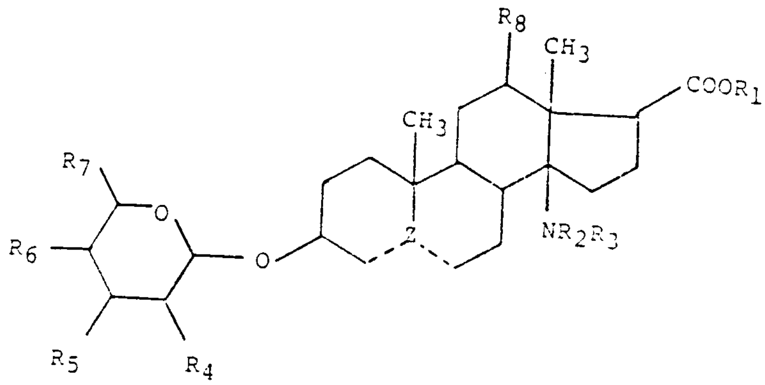

dans laquelle $R_1$ représente un groupe alkyle inférieur comportant 1 à 4 atomes de carbone, un groupe alkyle inférieur de 2 à 4 atomes de carbone substitué par un groupe amino, ou un groupe aralkyle de 6 à 12 atomes de carbone, $R_2$ et $R_3$, identiques ou différents, représentent un atome d'hydrogène ou un groupe alkyle inférieur comportant 1 à 4 atomes de carbone, $R_4$ représente un atome d'hydrogène, un groupe hydroxy, ou un groupe acétoxy, $R_5$ repésente un groupe hydroxy ou acétoxy, ou $R_4$ et $R_5$ représentent ensemple un groupe divalent alkylènedioxy, $R_6$ représente un groupe hydroxy, un groupe méthoxy ou un groupe acétoxy, $R_7$ représente un groupe méthyle ou un groupe hydroxyméthyle, $R_8$ représente un atome d'hydrogène ou un groupe hydroxy et --Z-- représente soit un groupe -CH-, soit un carbone =C- dont la double liaison est en position 4 ou 5 du noyau stéroïde.

**2.** Esters d'acide androstane 17-carboxylique selon la revendication 1, caractérisés en ce que $R_1$ est groupe méthyle ou un groupe éthyle.

**3.** Esters d'acide androstane 17-carboxylique selon l'une quelconque des revendications 1 et 2, caractérisés en ce que $R_5$ et $R_6$ représentent un groupe hydroxy.

**4.** Esters d'acide androstane 17-carboxylique selon l'une quelconque des revendications 1 et 2, caractérisés en ce que $R_4$ et $R_5$ se combinant pour former un groupe alkylènedioxy contenant 1 à 4 atomes de carbone dans la partie alkylène.

**5.** Esters d'acide androstane 17-carboxylique selon l'une quelconque des revendications 1 à 3, caractérisés en ce que $R_4$ représente un groupe hydroxy.

**6.** Esters d'acide androstane 17-carboxylique selon l'une quelconque des revendications précédentes, caractérisés en ce que le group amino en position 14, le groupe ester en position 17 et le reste sucré en position 3, ont la configuration $\beta$.

**7.** Esters d'acide androstane 17-carboxylique selon la revendication 1, charactérisés en ce qu'ils sont choisis parmi l'α-L-rhamnopyranosyloxy-3$\beta$ amino-14$\beta$ étianate de méthyle, l'α-L-rhamnopyranosyloxy-3$\beta$ méthylamino-14$\beta$ étianate de méthyle, et l'α-L-rhamnopyranosyloxy-3$\beta$ amino-14$\beta$ etièn-4 ate de méthyle.

**8.** Procédé de préparation des esters d'acide androstane 17-carboxylique selon la revendication 1, caractérisé en ce qu'on réalise une réaction de couplage d'un ester d'acide 3-hydroxy 14-azido androstane 17-carboxylique représenté par la formule générale (II) ci-dessous:

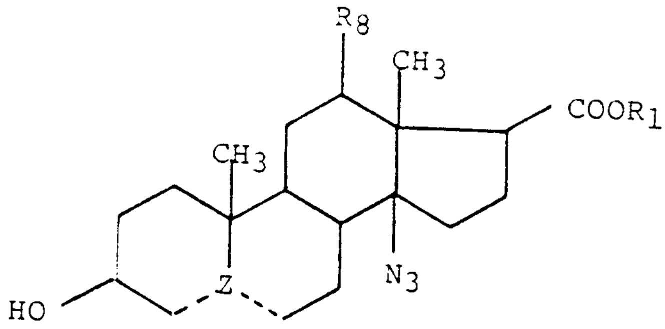

(II)

dans laquelle $R_1$ et $R_8$ ont la même signification que dans la formule générale (I) de la revendication 1, avec un dérivé activé de sucre, pour former un ester d'acide 3-pyranosyloxy 14-azido androstane 17-carboxylique de formule générale (III) ci-après:

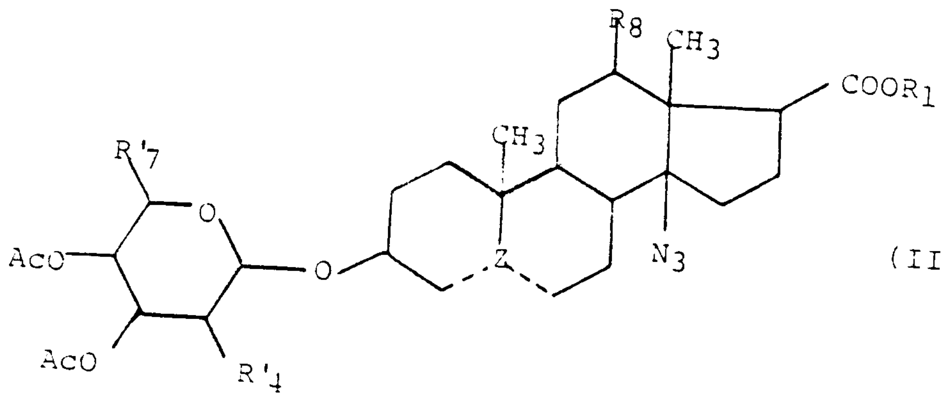

(III)

dans laquelle $R_4$' représente un atome d'hydrogène ou un groupe acétoxy, $R_7$' représente un group méthyle ou un group $-CH_2OAc$ et $R_8$ possède la même signification que dans la formule générale (I), que l'on réduit en dérivé de formule générale (I), puis on effectue si nécessaire une aminométhylation.

**9.** Procédé selon la revendication 8, caractérisé en ce que l'on effectue la réaction de couplage de l'ester d'acide 3-hydroxy 14-azido androstane 17-carboxylique avec le bromure de rhamnosyle en présence de cyanure mercurique.

**10.** Procédé selon la revendication 8, caractérisé en ce que l'on effectue la réaction de couplage de l'ester d'acide 3-hydroxy 14-azido androstane 17-carboxylique avec le tri-O-acétyl digitoxose en présence d'acide toluène sulfonique.

**11.** Procédé selon l'une quelconque des revendications 8 à 10, caractérisé en ce que la réduction de l'ester d'acide 3-pyranosyloxy 14-azido androstane 17-carboxylique de formule générale (III) est réalisée par action du borohydrure de sodium en présence de tellure dans un alcool, suivie si nécessaire d'une désacétylation.

**12.** Procédé selon l'une quelconque des revendications 8 à 10, caractérisé en ce que la réduction de l'ester d'acide 3-pyranosyloxy 14-azido androstane 17-carboxylique de formule générale (III) est réalisée par réaction d'hydrogénation en présence de nickel Raney, précédée si nécessaire d'une désacétylation.

**13.** Composition pharmaceutique, caractérisée en ce que qu'elle contient un ester d'acide androstane 17-carboxylique selon l'une quelconque des revendications 1 à 7, à titre de principe actif, associé le cas échéant à un ou plusieurs excipients pharmaceutiquement acceptables.

**14.** Utilisation d'un ester d'acide androstane 17-carboxylique selon l'une guelconque des revendications 1 à 7, pour la fabrication d'un médicament destiné a traitement de l'insuffisance cardiaque.

**Claims**

**1.** Androstane-17-carboxylic acid esters represented by general formula (I)

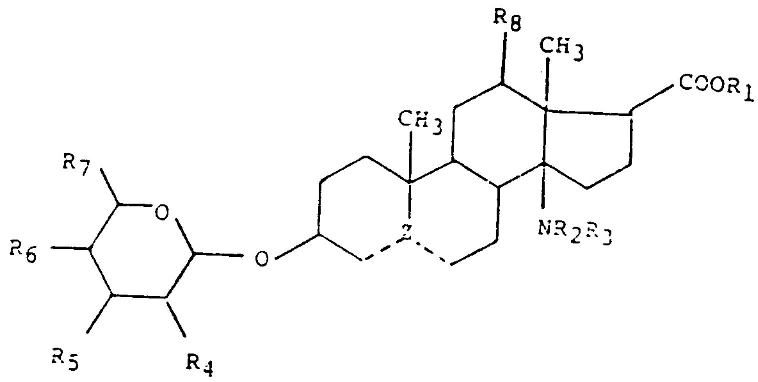

wherein $R_1$ represents a lower alkyl group containing 1 to 4 carbon atoms, a lower alkyl group from 2 to 4 carbon atoms substituted by an amino group, or an aralkyl group from 6 to 12 carbon atoms, $R_2$ and $R_3$, which may be the same or different, represent a hydrogen atom or a lower alkyl group containing 1 to 4 carbon atoms, $R_4$ represents a hydrogen atom, a hydroxy group, or an acetoxy group, $R_5$ represents a hydroxy group or an acetoxy group, or $R_4$ and $R_5$ represent together a divalent alkylenedioxy group, $R_6$ represents a hydroxy group, a methoxy group or an acetoxy group, $R_7$ represents a methyl group or a hydroxymethyl group, $R_8$ represents a hydrogen atom or a hydroxy group and -Z- represents either a -CH- group, or a =C- carbon where the double linkage is at the 4- or 5-position of the steroid nucleus.

**2.** Androstane-17-carboxylic acid esters according to claim 1, characterized in that $R_1$ is a methyl group or an ethyl group.

**3.** Androstane-17-carboxylic acid esters according to any of claims 1-2, characterized in that $R_5$ and $R_6$ represent a hydroxy group.

**4.** Androstane-17-carboxylic acid esters according to any of claims 1-2, characterized in that $R_4$ and $R_5$ are combined to form an alkylenedioxy group containing 1 to 4 carbon atoms in the alkylene moiety thereof.

**5.** Androstane-17-carboxylic acid esters according to any of claims 1 to 3, characterized in that $R_4$ represents a hydroxy group.

**6.** Androstane-17-carboxylic acid esters according to any of the preceding claims, characterized in that the amino group at the 14-position, the ester group at the 17-position, and the sugar residue at the 3-position, have the ß configuration.

**7.** Androstane-17-carboxylic acid esters according to claim 1, characterized in that they are selected from methyl α-L-rhamnopyranosyloxy-3ß amino-14ß etianate, methyl α-L-rhamnopyranosyloxy-3ß methylamino-14ß etianate and methyl α-L-rhamnopyranosyloxy-3ß amino-14ß etien-4-ate.

**8.** A process for the preparation of the androstane-17-carboxylic acid esters of claim 1, characterized in that it comprises carrying out a coupling reaction of a 3-hydroxy-14-azido-androstane-17-carboxylic acid ester, represented by the following general formula (II) :

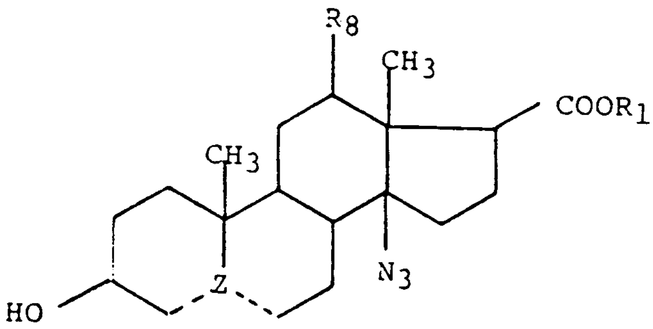

(II)

wherein $R_1$ and $R_8$ are as defined in the general formula (I) of claim 1, with an activated sugar residue, thus forming a 3-pyranosyloxy-14-azido-androstane-17-carboxylic acid ester of the following general formula (III) :

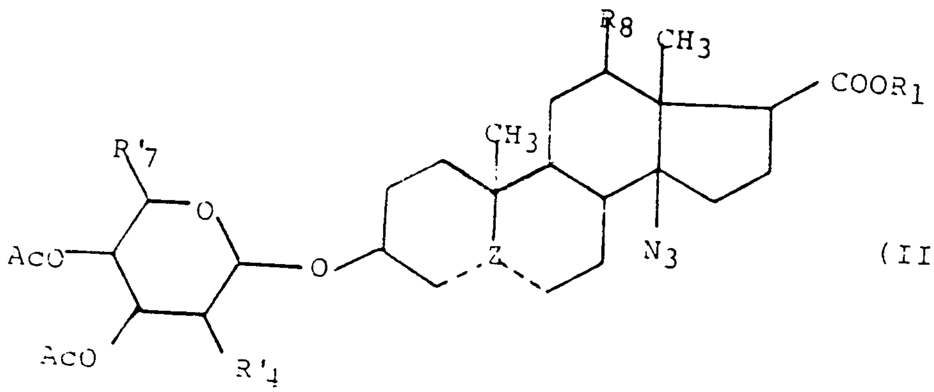

(III)

wherein $R'_4$ represents a hydrogen atom or an acetoxy group, and $R'_7$ represents a methyl group or a $-CH_2OAc$ group, an $R_8$ is as defined in the general formula (I), which is reduced into the derivative of general formula (I), followed if necessary by an aminomethylation.

**9.** A process according to claim 8, characterized in that it comprises carrying out a coupling reaction between the 3-hydroxy-14-azido-androstane-17-carboxylic acid ester and rhamnosyl bromide in the presence of mercury cyanide.

**10.** A process according to claim 8, characterized in that it comprises carrying out a coupling reaction between the 3-hydroxy-14-azido-androstane-17-carboxylic acid ester and tri-O-acetyl-digitoxose in the presence of toluene sulfonic acid.

**11.** A process according to any of claims 8 to 10, characterized in that the reduction of 3-pyranosyloxy-14-azido-androstane-17-carboxylicacid ester of general formula (III) is carried out by action of sodium borohydride in the presence of tellure in an alcohol, followed if necessary by a deacetylation.

**12.** A process according to any of claims 8 to 10, characterized in that the reduction of 3-pyranosyloxy-14-azido-androstane-17-carboxylicacid ester of general formula (III) is carried out by hydrogenation reaction in the presence of Raney nickel, preceded if necessary by a deacetylation.

**13.** A pharmaceutical composition, characterized in that it comprises an androstane-17-carboxylic acid ester according to any of claims 1 to 7, as an active principle, combined if necessary with one or several pharmaceutically acceptable carriers.

**14.** The use of an androstane-17-carboxylic acid ester according to any of claims 1 to 7, for the manufacture of a medicament for the treatment of heart failure.

## Patentansprüche

1. Androstan-17-carbonsäureester, dargestellt durch die allgemeine Formel (I):

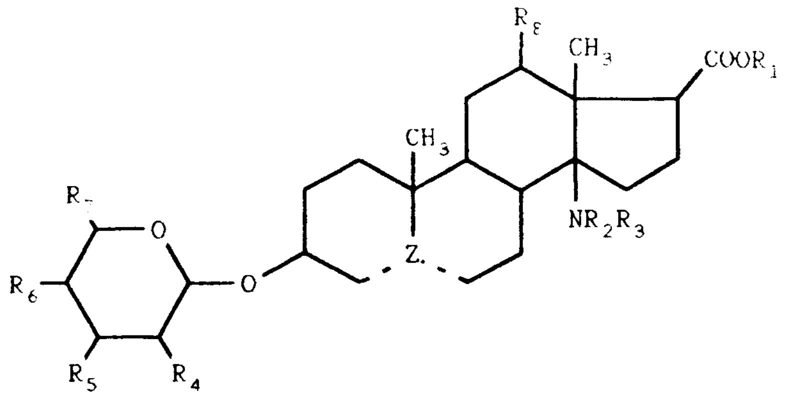

(I)

in welcher $R_1$ für eine 1 bis 4 Kohlenstoff-Atome enthaltende Niederalkyl-Gruppe, eine durch eine Amino-Gruppe substituierte Niederalkyl-Gruppe mit 2 bis 4 Kohlenstoff-Atomen oder eine Aralkyl-Gruppe mit 6 bis 12 Kohlenstoff-Atomen steht, $R_2$ und $R_3$ gleich oder verschieden sind und für ein Wasserstoff-Atom oder eine 1 bis 4 Kohlenstoff-Atome enthaltende Niederalkyl-Gruppe stehen, $R_4$ für ein Wasserstoff-Atom, eine Hydroxy-Gruppe oder eine Acetoxy-Gruppe steht, $R_5$ für eine Hydroxy- oder Acetoxy-Gruppe steht oder $R_4$ und $R_5$ zusammen für eine zweiwertige Alkylendioxy-Gruppe stehen, $R_6$ für eine Hydroxy-Gruppe, eine Methoxy-Gruppe oder eine Acetoxy-Gruppe steht, $R_7$ für eine Methyl-Gruppe oder eine Hydroxymethyl-Gruppe steht, $R_8$ für ein Wasserstoff-Atom oder eine Hydroxy-Gruppe steht und --Z-- entweder für eine -CH-Gruppe oder einen Kohlenstoff =C-, dessen Doppelbindung sich in 4- oder 5-Stellung des Steroidgerüstes befindet, steht.

2. Androstan-17-carbonsäureester gemäß Anspruch 1, dadurch gekennzeichnet, daß $R_1$ eine Methyl-Gruppe oder eine Ethyl-Gruppe ist.

3. Androstan-17-carbonsäureester gemäß einem der Ansprüche 1 und 2, dadurch gekennzeichnet, daß $R_5$ und $R_6$ für eine Hydroxy-Gruppe stehen.

4. Androstan-17-carbonsäureester gemäß einem der Ansprüche 1 und 2, dadurch gekennzeichnet, daß $R_4$ und $R_5$ sich verbinden, um eine im Alkylen-Teil 1 bis 4 Kohlenstoff-Atome enthaltende Alkylendioxy-Gruppe zu bilden.

5. Androstan-17-carbonsäureester gemäß einem der Ansprüche 1 bis 3, dadurch gekennzeichnet, daß $R_4$ für eine Hydroxy-Gruppe steht.

6. Androstan-17-carbonsäureester gemäß einem der voranstehenden Ansprüche, dadurch gekennzeichnet, daß die Amino-Gruppe in 14-Stellung, die Ester-Gruppe in 17-Stellung und der Zucker-Rest in 3-Stellung die ß-Konfiguration haben.

7. Androstan-17-carbonsäureester gemäß Anspruch 1, dadurch gekennzeichnet, daß sie unter Methyl-α-L-rhamnopyranosyloxy-3ß-amino-14ß-ätianat, Methyl-α-L-rhamnopyranosyloxy-3ß-methylamino-14ß-ätianatund Methyl-α-L-rhamnopyranosyloxy3ß-amino-14ß-äti-4-enat ausgewählt sind.

8. Verfahren zur Herstellung von Androstan-17-carbonsäureestern gemäß Anspruch 1, dadurch gekennzeichnet, daß man eine Kupplungsreaktion eines durch die nachstehende allgemeine Formel (II)

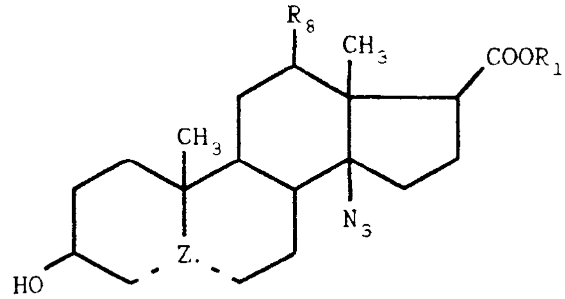

(II)

in welcher $R_1$ und $R_8$ dieselbe Bedeutung wie in der allgemeinen Formel (I) von Anspruch 1 haben, wiedergegebenen 3-Hydroxy-14-azidoandrostan-17-carbonsäureesters mit einem aktivierten Zucker-Derivat durchführt, um einen 3-Pyranosyloxy-14-azidoandrostan-17-carbonsäureester der nachstehenden allgemeinen Formel (III)

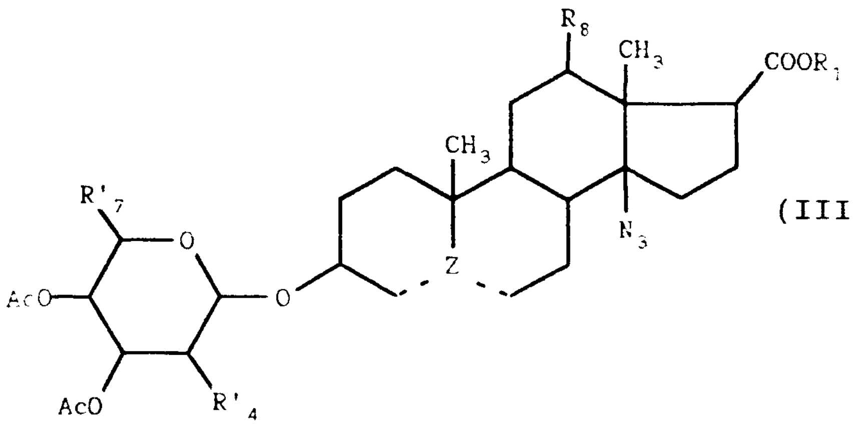

(III)

in welcher $R'_4$ für ein Wasserstoff-Atom oder eine Acetoxy-Gruppe steht, $R'_7$ für eine Methyl-Gruppe oder eine $-CH_2OAc$-Gruppe steht und $R_8$ dieselbe Bedeutung wie in der allgemeinen Formel (I) besitzt, zu erhalten, welchen man zu einem Derivat der allgemeinen Formel (I) reduziert und dann, falls erforderlich, eine Aminomethylierung durchführt.

**9.** Verfahren gemäß Anspruch 8, dadurch gekennzeichnet, daß man die Kupplungsreaktion des 3-Hydroxy-14-azidoandrostan-17-carbonsäureesters mit Rhamnosylbromid in Gegenwart von Quecksilbercyanid durchführt.

**10.** Verfahren gemäß Anspruch 8, dadurch gekennzeichnet, daß man die Kupplungsreaktion des 3-Hydroxy-14-azidoandrostan-17-carbonsaureesters mit Tri-O-acetyldigitoxose in Gegenwart von Toluolsulfonsäure durchführt.

**11.** Verfahren gemäß einem der Ansprüche 8 bis 10, dadurch gekennzeichnet, daß man die Reduktion des 3-Pyranosyloxy-14-azidoandrostan-17-carbonsaureesters der allgemeinen Formel (III) durch Einwirkung von Natriumborhydrid in Gegenwart von Tellur in einem Alkohol, erforderlichenfalls gefolgt von einer Deacetylierung, bewerkstelligt.

**12.** Verfahren gemäß einem der Ansprüche 8 bis 10, dadurch gekennzeichnet, daß man die Reduktion des 3-Pyranosyloxy-14-azidoandrostan-17-carbonsäureesters der allgemeinen Formel (III) durch eine Hydrierungs-Reaktion in Gegenwart von Raney-Nickel in einem Alkohol, erforderlichenfalls gefolgt von einer Deacetylierung, bewerkstelligt.

**13.** Pharmazeutische Zusammensetzung, dadurch gekennzeichnet, daß sie als Wirkstoff einen Androstan-17-carbonsäureester gemäß einem der Ansprüche 1 bis 7, welcher gegebenenfalls von einem oder mehreren pharmazeutisch annehmbaren Hilfsstoffen begleitet wird, enthält.

**14.** Verwendung eines Androstan-17-carbonsäureesters gemäß einem der Ansprüche 1 bis 7 zur Herstellung eines Medikaments zur Behandlung von Herzinsuffizienz.